# EUROPÄISCHE PATENTANMELDUNG

(11) **EP 1 844 753 A2**
(43) Veröffentlichungstag der Anmeldung: **17.10.2007**
(21) Anmeldenummer: 07005866.4
(22) Anmeldetag: 22.03.2007
(51) Int. Cl.: A61K 8/04, A61K 8/06, A61K 8/41, A61Q 17/04

(54) **Verwendung von Esterquats in Zusammensetzungen als sandabweisende Substanzen**

(30) Priorität: 04.04.2006 DE 102006015753
(71) Anmelder: Goldschmidt GmbH, 45127 Essen (DE)
(72) Erfinder: Grüning, Burghard, 45134 Essen (DE); Jenni, Klaus, 45357 Essen (DE); Mathiak, Ralf, 45968 Gladbeck (DE); Meyer, Jürgen, 48143 Münster (DE); Weitemeyer, Christian, 45257 Essen (DE)

(57) **Zusammenfassung**

Der Gegenstand der vorliegenden Erfindung betrifft die Verwendung von Esterquats in Zusammensetzungen, wobei die Esterquats für die Zusammensetzungen, umfassend kosmetische Zusammensetzungen, eine sandabweisende Wirkung hervorrufen, sowie die Verwendung dieser kosmetischen Zusammensetzungen mit sandabweisender Wirkung zur topischen Anwendung, beispielsweise für den Schutz der Haut vor Ultraviolett-Strahlung. Der Gegenstand der vorliegenden Patentanmeldung betrifft insbesondere die Verwendung wenigstens eines Esterquats auf Basis von Alkanolaminen in Zusammensetzungen, wobei die Esterquats für die Zusammensetzungen eine sandabweisende Wirkung hervorrufen, und die Acylkomponente der Esterquats sich von
(a) Monocarbonsäuren,
(b) Dicarbonsäuren,
(c) Tricarbonsäuren,
oder deren Gemischen ableitet.

## Beschreibung

Die vorliegende Erfindung betrifft die Verwendung von Esterquats auf Basis von Alkanolaminen als sandabweisende Substanzen in Zusammensetzungen sowie kosmetische Zusammensetzungen, enthaltend Esterquats als sandabweisende Substanzen, sowie die Verwendung dieser kosmetischen Zusammensetzungen zur topischen Verwendung mit sandabweisender Wirkung, insbesondere für den Schutz der Haut vor Ultraviolett-Strahlung.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut. Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Etwa 90 % der auf die Erde gelangenden ultravioletten Strahlung besteht aus UV-A-Strahlen. Während die UV-B-Strahlung in Abhängigkeit von zahlreichen Faktoren, wie Jahres- und Tageszeit oder Breitengrad, stark variiert, bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Es ist daher von grundsätzlicher Wichtigkeit, dass kosmetische Lichtschutzzubereitungen sowohl gegen UV-B- als auch gegen UV-A-Strahlung ausreichenden Schutz bieten.

Der Stand der Technik kennt ferner Produkte zur Pflege der Haut, welche nach dem Sonnenbaden angewendet werden und üblicherweise spezielle Wirkstoffe enthalten, wie beispielsweise Rückfettungs- und Feuchthaltemittel, entzündungslindernde und kühlende Stoffe, lokal anästhesierende Stoffe und/oder desinfizierende Stoffe, um beispielsweise mögliche Hautinfektionen zu verhindern. Diese so genannten Aftersun-oder Apres-soleil-Präparate sind dazu bestimmt, die Haut nach dem Sonnenbad zu kühlen und ihr Feuchthaltevermögen zu verbessern, wobei die Vermittlung des Kühleffektes eine zentrale Rolle spielt. Allerdings mangelt es dem Stand der Technik an Produkten, welche die Haut bereits während der UV-Einstrahlung vor dem Austrocknen schützen und sie ausreichend pflegen.

Ein Nachteil des Standes der Technik ist, dass übliche Lichtschutzformulierungen sowie Produkte zur Pflege der Haut einen meist klebrigen Film auf der Haut hinterlassen. Dies hat bei der Anwendung solcher Produkte an einem Sandstrand zur Folge, dass der Sand am Körper haften bleibt, was vom Anwender als unangenehm empfunden wird und dazu führen kann, dass die Lichtschutzformulierungen sowie Produkte zur Pflege der Haut zu wenig oder gar nicht mehr verwendet werden. Da am Strand meist ein mehr oder weniger starker Wind herrscht, tritt dieser Nachteil in der Regel selbst dann auf, wenn der Körper gar nicht direkt mit dem Sand in Berührung kommt, beispielsweise beim Sonnenbaden im Strandkorb oder Liegestuhl, da auch der im Wind herumwirbelnde Sandstaub auf den eingecremten Hautpartien haften bleibt.

Eine Aufgabe der vorliegenden Erfindung war daher, neue Substanzen und diese enthaltende kosmetische Zusammensetzungen, insbesondere Lichtschutzformulierungen, zu finden, nach deren Anwendung kein Sand auf der eingecremten Haut kleben bleibt oder die Sandanhaftung zumindest deutlich reduziert wird. Substanzen, die eine solche gewünschte Eigenschaft aufweisen, werden nachstehend als sandabweisend bezeichnet.

Es war überraschend und für den Fachmann nicht vorauszusehen, dass in Zusammensetzungen die Verwendung von Esterquats auf Basis von Alkanolaminen für die Zusammensetzung eine sandabweisende Wirkung hervorruft, wobei sich die Acylkomponente des Esterquats von
(a) Monocarbonsäuren,
(b) Dicarbonsäuren,
(c) Tricarbonsäuren,
oder deren Gemischen ableitet.

Die erfindungsgemäßen Esterquat/s enthaltenden Zusammensetzungen mit sandabweisender Wirkung können Ethanolamin, insbesondere Triethanolamin, Isopropanolamin, insbesondere Triisopropanolamin, mit einem Gewichtsgehalt von ≥ 0 Gew.-% bis ≤ 5 Gew.-%, gegebenenfalls ≥ 0,01 Gew.-% bis ≤ 3 Gew.%, ≥ 0, 1 Gew.-% bis ≤ 2 Gew.-%, enthalten. Es kann bevorzugt sein, dass der Gehalt an freiem Triethanolamin in der Esterquat enthaltenden Zusammensetzung maximal 1000 ppm ausmacht.

Zur Herstellung der Esterquats können erfindungsgemäß Alkanolamine verwendet werden, die ausgewählt sind aus der Gruppe umfassend Mono-, Di- und/oder Tri-Alkanolamine. Der Alkanolrest kann jeweils gleich oder unabhängig voneinander ein verzweigter oder unverzweigter, gesättigter oder ungesättigter Hydroxy-Rest mit C₁-C₆ Kohlenstoffatomen, vorzugsweise verzweigtes oder unverzweigtes C₃-C₅-Hydroxyalkyl, verzweigtes oder unverzweigtes C₃-C₅-Hydroxyalkenyl, und besonders bevorzugt Hydroxyethyl oder Hydroxy-isopropyl, sein.

Unter der Bezeichnung "Esterquats auf Basis von Alkanolaminen", in der vorliegenden Erfindung auch als "Esterquats" bezeichnet, werden im allgemeinen quaternierte Fettsäurealkanolaminester sowie Salze davon verstanden.

Es handelt sich dabei um bekannte Stoffe, die man nach den einschlägigen Methoden der präparativen organischen Chemie erhalten kann. Die Herstellung von Esterquats wird beispielsweise in der WO 91/01295 beschrieben, nach der man Triethanolamin in Gegenwart von unterphosphoriger Säure mit Fettsäuren partiell verestert, Luft durchleitet und anschließend mit Dimethylsulfat quaterniert. Auch aus der Deutschen Patentschrift DE 4308794 C1 ist ein Verfahren zur Herstellung fester Esterquats bekannt, bei dem man die Quaternierung von Triethanolaminestern in Gegenwart von geeigneten Fettalkoholen durchführt. Bei einem weiteren bekannten Verfahren zur Herstellung von Esterquats kann sowohl von Fettsäuren, als auch den entsprechenden Triglyceriden in Abmischung mit Dicarbonsäuren ausgegangen werden. Ein solches Herstellungsverfahren ist in der europäischen Patentschrift EP 0 750 606 B1 beschrieben

Unter der Bezeichnung "Esterquat mit sandabweisender Wirkung" wird verstanden, dass die erfindungsgemäße Verwendung der Esterquats in Zusammensetzungen diesen eine sandabweisende Wirkung verleiht, d.h. sie führt beispielsweise zu einer Reduzierung der Sandanhaftung an der Haut.

Überraschenderweise wurde nun gefunden, dass Esterquats in Zusammensetzungen eine sandabweisende Wirkung besitzen, so dass deren Verwendung in kosmetischen Zusammensetzungen, umfassend Lichtschutzformulierungen, sowie Produkten zur Pflege der Haut, aufgrund der verringerten Sandanhaftung das Hautgefühl verbessern.

Die erfindungsgemäßen kosmetischen Lichtschutzformulierungen können zur Behandlung, Pflege und Reinigung der Haut und als Schminkprodukt in der dekorativen Kosmetik dienen. Lichtschutzformulierungen werden nachfolgend auch als Sonnenschutzmittel bezeichnet. Entsprechend ihrem Aufbau können kosmetische Zusammensetzungen im Sinne der vorliegenden Erfindung beispielsweise verwendet werden als Hautschutzcreme, Reinigungsmilch, Tages- oder Nachtcreme usw. Es ist gegebenenfalls möglich und vorteilhaft, die erfindungsgemäßen Zusammensetzungen als Grundlage für pharmazeutische Formulierungen zu verwenden. Zur Anwendung werden die kosmetischen Zusammensetzungen in der für Kosmetika üblichen Weise auf die Haut in ausreichender Menge aufgebracht.

Kosmetische Zusammensetzungen mit sandabweisender Wirkung im Sinne dieser Erfindung umfassen aber auch dermatologische Zusammensetzungen. Erfindungsgemäße kosmetische Zusammensetzungen, nachfolgend auch als dermatologische Zusammensetzungen bezeichnet, deren hauptsächlicher Zweck nicht der Schutz vor Sonnenlicht ist, können trotzdem einen Gehalt an UV-Schutzsubstanzen aufweisen. Bekanntermaßen können Tages-Cremes oder Make-up-Produkte UV-A- und/oder UV-B-Filtersubstanzen aufweisen.
Die erfindungsgemäßen Esterquats und Esterquats enthaltende Zusammensetzungen zeigen sehr gute sensorische und kosmetische Eigenschaften, insbesondere eine gute Verteilbarkeit auf der Haut. Noch ein Vorteil der vorliegenden Erfindung ist, dass bei den vorliegenden erfindungsgemäßen Zusammensetzungen, enthaltend Esterquats zur Erzeugung einer sandabweisenden Wirkung der Zusammensetzung, neben den Esterquat/s gegebenenfalls auf den Zusatz von zusätzlichen Emulgatoren verzichtet werden kann.

Insbesondere bei Allergikern ist es wichtig, die Anzahl unterschiedlicher Komponenten möglichst gering zu halten, um eine gute Hautverträglichkeit zu gewährleisten. Da Esterquats bekanntermaßen eine biozide Wirkung aufweisen und man gegebenenfalls auf den Zusatz von Emulgatoren, wie vorstehend beschrieben, verzichten kann, ist es möglich, Lichtschutzformulierungen sowie Produkte zur Linderung und Pflege der Haut mit einer verringerten Anzahl von Komponenten zur Verfügung zu stellen.

Erfindungsgemäße, geeignete Mono-, Di- und Tri-Esterquats, die im Sinne der Erfindung Verwendung finden können, sind Produkte, deren Acylkomponente sich von Monocarbonsäuren der Formel RCO-OH ableiten, in der R für einen linearen oder verzweigten, gesättigten oder ungesättigten, gegebenenfalls hydroxysubstituierten Acylrest mit 5 bis 23 Kohlenstoffatomen steht. Erfindungsgemäß geeignete Carbonsäuren sind Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, die man beispielsweise bei der Druckspaltung von natürlichen Fetten und Ölen erhält.

Des weiteren können in erfindungsgemäßen Zusammensetzungen Esterquats eine sandabweisende Wirkung hervorrufen, bei denen wenigstens eine Acylkomponente des Esterquats sich von Dicarbonsäuren der Formel HOOC(CH₂) ₙCOOH ableiten, in der n für Zahlen von 1 bis 10 steht. Geeignete Dicarbonsäuren sind beispielsweise Malonsäure, Bernsteinsäure, Maleinsäure, Fumarsäure, Glutarsäure, Sorbinsäure, Pimelinsäure, Azelainsäure, Sebacinsäure und/oder Dodecandisäure.

Erfindungsgemäß bevorzugt ist die Verwendung von wenigstens einem sandabweisenden Esterquat, wobei Gemische bevorzugt sind, welche die nachstehende Formel I, Formel II und/oder Formel III aufweisen: 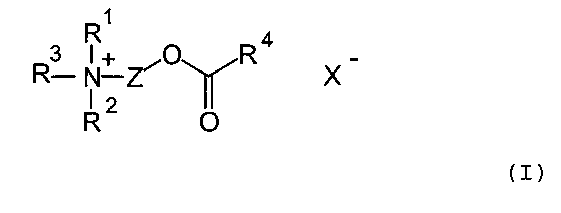 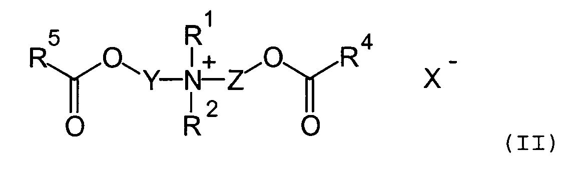 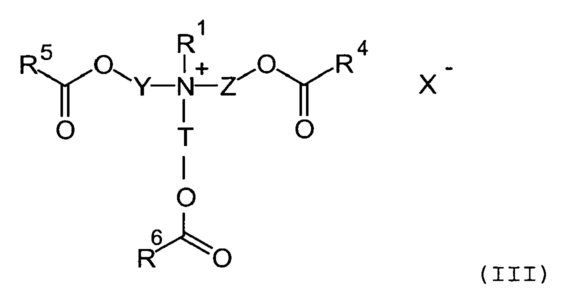 in der bedeuten:
- R¹ =: H, Methyl, Ethyl, Propyl oder iso-Propyl, vorzugsweise Methyl oder Ethyl,
- R², R³ =: gleich oder unabhängig voneinander, R¹, verzweigter oder unverzweigter, gesättigter oder ungesättigter Hydroxyalkyl-Rest mit C₁-C₆ Kohlenstoffatomen, vorzugsweise verzweigtes oder unverzweigtes C₃-C₅-Hydroxyalkyl, verzweigtes oder unverzweigtes C₃-C₅-Hydroxyalkenyl, und besonders bevorzugt Hydroxyethyl oder Hydroxyisopropyl,
- R⁴ R⁵ und R⁶ =: gleich oder unabhängig voneinander, verzweigter oder unverzweigter, gesättigter oder ungesättigter Acyl-Rest mit C₅-C₂₃ Kohlenstoffatomen, vorzugsweise mit C₇-C₂₁ Kohlenstoffatomen, besonders bevorzugt mit C₁₁-C₁₉ Kohlenstoffatomen und am meisten bevorzugt mit C₁₅-C₁₇ Kohlenstoffatomen,
- T, Y und Z =: gleich oder unabhängig voneinander, Methylen, Ethylen, Ethenylen, verzweigtes oder unverzweigtes, gesättigtes oder ungesättigtes Alkylen mit C₃-C₅-Kohlenstoffatomen, und
- X =: Anion, vorzugsweise ist das Anion ein Halogenid, Alkylsulfat oder Alkylphosphat, und besonders bevorzugt ist das Anion Cl⁻, CH₃OSO₃⁻ oder CH₃CH₂OSO₃⁻.

Gemäß einer besonders bevorzugten Ausführungsform der vorliegenden Erfindung können sandabweisende Esterquats verwendet werden, die ausgewählt sind aus der Gruppe umfassend Di-(oleylcarboxyethyl)-hydroxyethyl-methylammoniumsalz, Di-(talgcarboxyethyl)-hydroxyethyl-methylammoniumsalz, N,N-Di-(ß-stearoylethyl)-N,N-dimethyl-ammoniumsalz, N,N-Di-(ß-palmitoylethyl)-N,N-dimethyl-ammoniumsalz, Dicocoylethylhydroxyethylammonium-methosulfate, Dipalmoylethylhydroxyethylammonium-methosulfate, Dirapscarboxyethylhydroxyethylammonium-methosulfate, und/oder Disoyacarboxyethyl-hydroxyethylammonium-methosulfate und/oder deren hydrierte Analoga.

Gemäß der vorliegenden Erfindung können die Esterquats mit sandabweisender Wirkung einzeln oder in Form von Gemischen verwendet werden. Im Hinblick auf die anwendungstechnischen Eigenschaften der sandabweisenden Esterquats hat sich für Mischungen von Esterquats ein durchschnittlicher Veresterungsgrad von 1 bis 3, vorzugsweise 1,5 bis 2,5, und bevorzugt 1,7 bis 2,2 als besonders vorteilhaft herausgestellt. Weiterhin ist die Verwendung von sandabweisenden Esterquats bevorzugt, bei denen es sich um technische Mischungen von Mono-, Di- und Triestern mit einem durchschnittlichen Veresterungsgrad von 1,5 bis 1,9 handelt.

Zur Einstellung der gewünschten Jodzahl können die Esterquats nach gängigen Verfahren hydriert werden.

So hat sich gezeigt, dass sandabweisende Esterquats mit einer hohen Jodzahl gegenüber Esterquats mit einer geringeren Jodzahl eine deutlich verbesserte sandabweisende Wirkung aufweisen können. Die Verwendung von sandabweisenden Esterquats, die eine Jodzahl von ≥ 20 bis ≤ 100, vorzugsweise eine Jodzahl von ≥ 30 bis ≤ 90, und besonders bevorzugt eine Jodzahl von ≥ 40 bis ≤ 80, aufweisen, kann deshalb erfindungsgemäß am meisten bevorzugt sein. Insbesondere Esterquats mit Jodzahlen im Bereich von ≥ 60 bis ≤ 95 sowie ≥ 70 bis ≤ 85 können eine gesteigerte sandabweisende Wirkung aufweisen. Die Jodzahl, wenn nicht anders angegeben, bezieht sich auf die jeweilige Esterquatverbindung als solche.

Es hat sich gezeigt, dass Esterquats einer Zusammensetzung eine gute sandabweisende Wirkung verleihen können, wenn die verwendeten Esterquats einen Veresterungsgrad von 1,5 bis 2,5 und vorzugsweise von 1,7 bis 2,2, sowie eine Jodzahl von 20 bis 60 und vorzugsweise eine Jodzahl von 30 bis 40 aufweisen. Besonders bevorzugt können Esterquats mit einem Veresterungsgrad von 1,75 und einer Jodzahl von 32 bis 36 eingesetzt werden.

Ein weiterer Gegenstand der vorliegenden Erfindung betrifft eine kosmetische Zusammensetzung, enthaltend Esterquats mit sandabweisender Wirkung, wobei die kosmetischen Zusammensetzungen Lichtschutzformulierungen sowie Produkte zur Linderung der Hautreizung und Pflege der Haut umfassen.

Bevorzugt betrifft der Gegenstand der vorliegenden Erfindung eine Zusammensetzung mit sandabweisender Wirkung, enthaltend Esterquats zum Schutz der menschlichen Epidermis oder ein Sonnenschutzmittel, sowie deren Verwendung, wobei die kosmetische Zusammensetzung mit sandabweisender Wirkung in Form einer nicht-ionischen Vesikeldispersion, einer Emulsion, insbesondere einer Emulsion vom Öl-in-Wasser-Typ, einer Creme, einer Milch, eines Gels, einer Gelcreme, einer Suspension, einer Dispersion, eines Puders, eines festen Stifts, eines Schaumes oder eines Sprays vorliegt.

Erfindungsgemäß geeignet ist auch eine kosmetische Zusammensetzung mit sandabweisender Wirkung enthaltend Esterquats sowie deren Verwendung zum Schminken der Wimpern, der Augenbrauen oder der Haut, wobei diese in fester oder pastöser, wasserfreier oder wässriger Form, in Form einer Emulsion, einer Suspension oder einer Dispersion vorliegen kann.

Erfindungsgemäß geeignete kosmetische Zusammensetzungen zur topischen Verwendung mit sandabweisender Wirkung, enthalten einen kosmetisch akzeptablen Träger und wenigstens ein Esterquat als sandabweisende Substanz.

Die kosmetischen Zusammensetzungen können Esterquat/s als sandabweisende Substanz mit einem Gewichtsgehalt, bezogen auf das Gesamtgewicht der kosmetischen Zusammensetzung, von 0.1 Gew.-% bis 10 Gew.-%, vorzugsweise von 1 Gew.-% bis 8 Gew.-%, und besonders bevorzugt von 2 Gew.-% bis 4 Gew.-%, aufweisen.

Die kosmetischen Zusammensetzungen im Sinne der vorliegenden Erfindung liegen bevorzugt in Form von O/W-Emulsionen vor. Insbesondere kann es bevorzugt sein, dass der kosmetisch akzeptable Träger in Form einer Emulsion vom Öl-in-Wasser-Typ vorliegt. Die Zusammensetzungen im Sinne der vorliegenden Erfindung können daher bevorzugt neben einer oder mehrerer Ölphasen zusätzlich eine oder mehrere Wasserphasen enthalten und beispielsweise in Form von O/W-, W/O/W- oder mehrfach multipler Emulsionen vorliegen. Solche Formulierungen können vorzugsweise auch eine Mikroemulsion, eine Feststoff-Emulsion, d. h. eine Emulsion, welche durch Feststoffe stabilisiert ist, z. B. eine Pickering-Emulsion, eine sprühbare Emulsion oder eine Hydrodispersion sein.

Die Ölphase kann beispielsweise ausgewählt sein aus der Gruppe der polaren Öle, umfassend Lecithine, Fettsäuretriglyceride, Triglycerinester gesättigter und/oder ungesättigter, verzweigter und/oder unverzweigter Alkancarbonsäuren einer Kettenlänge von 8 bis 24, insbesondere 12 bis 18 C-Atomen.

Die Fettsäuretriglyceride können ausgewählt sein aus der Gruppe der synthetischen, halbsynthetischen und/oder natürlichen Öle, umfassend Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl und dergleichen.

Weiterhin sind natürliche Wachse tierischen und pflanzlichen Ursprungs verwendbar, umfassend Bienenwachs und andere Insektenwachse, vorzugsweise Beerenwachs, Sheabutter und/oder Lanolin.

Polare Ölkomponenten können ausgewählt werden aus der Gruppe der Ester, umfassend gesättigte und/oder ungesättigte, verzweigte und/oder unverzweigte Alkancarbonsäuren mit einer Kettenlänge von 3 bis 30 C- Atomen, gesättigte und/oder ungesättigte, verzweigte und/oder unverzweigte Alkohole mit einer Kettenlänge von 3 bis 30 C-Atomen, Ester aus aromatischen Carbonsäuren, gesättigte und/oder ungesättigte, verzweigte und/oder unverzweigte Alkohole einer Kettenlänge von 3 bis 30 C-Atomen.

Bevorzugte Esteröle umfassen Octylpalmitat, Octylcocoat, Octylisostearat, Octyldodecylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexylcocoat, 2-Ethylhexylisostearat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, insbesondere Jojobaöl.

Die Ölphase kann aber auch Dialkylether und Dialkylcarbonate, Dicaprylylether und/oder Dicaprylylcarbonat, Diethylhexylether und/oder Diethylhexylcarbonat umfassen.

Geeignete Ölkomponenten können ausgewählt sein aus der Gruppe umfassend Isoeikosan, Neopentylglykoldiheptanoat, Propylenglykoldicaprylat/dicaprat, Caprlic/Capric/Diglycerylsuccinat, Butylenglykol Dicaprylat/Dicaprat, C12-13-Alkyllactat, Di-C 12-13-Alkyltartrat, Triisostearin, Dipentaerythrityl Hexacaprylat/Hexacaprat, Propylenglykolmonoisostearat, Tricaprylin, Dimethylisosorbid, C12-15-Al-kylbenzoat, Butyloctylsalicylat, Hexadecylbenzoat und Butyloctylbenzoat und Gemische davon.

Als Ölphase lassen sich auch unpolare Öle verwenden, umfassend verzweigte und unverzweigte Kohlenwasserstoffe und - wachse, insbesondere Mineralöl, Vaseline, Paraffinöl, Squalan und Squalen, Polyolefine, hydrogenierte Polyisobutene und Isohexadecan.

Die Ölphase kann ferner einen Gehalt an cyclischen oder linearen Silikonölen aufweisen oder vollständig aus solchen Ölen bestehen. Systematisch werden die Silikonöle als Polyorganosiloxane bezeichnet. Die methylsubstituierten Polyorganosiloxane, welche die mengenmäßig bedeutendsten Verbindungen dieser Gruppe darstellen, werden auch als Polydimethylsiloxan bzw. Dimethicone (INCI) bezeichnet. Dimethicone gibt es in verschiedenen Kettenlängen bzw. mit verschiedenen Molekulargewichten.

Geeignete Polyorganosiloxane gemäß der vorliegenden Erfindung umfassen Dimethylpolysiloxane, welche beispielsweise unter den Handelsbezeichnungen Abil® 10 bis 10 000 bei der Firma Goldschmidt GmbH erhältlich sind, Cetyldimethicone, Hexamethylcyclotrisiloxan, Polydimethylsiloxan, Poly-(methylphenylsiloxan), Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Dimethicone), welche als verschiedene Abil® Wax-Typen bei der Firma Goldschmidt GmbH erhältlich sind.

Die kosmetischen Zusammensetzungen können, soweit es sich um Sonnenschutzmittel handelt, UV-A- und/oder UV-B-Filtersubstanzen aufweisen. Vorteilhaft enthalten die erfindungsgemäßen Zusammensetzungen die Substanzen, die UV-Strahlung im UV-A- und/oder UV-B-Bereich absorbieren, in einer Gesamtmenge von 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zusammensetzungen, um kosmetische Zusammensetzungen zur Verfügung zu stellen, die die Haut vor dem gesamten Bereich der ultravioletten Strahlung schützen.

Die erfindungsgemäßen kosmetischen Zusammensetzungen, insbesondere Sonnenschutzmittel, können ein oder mehrere ergänzende hydrophile oder lipophile, im UV-A- und/oder UV-B-Bereich wirksame Filter enthalten.

Geeignete organische Filter sind beispielsweise Zimtsäurederivate, Salicylsäurederivate, Campherderivate, Triazinderivate, Benzophenonderivate, Dibenzoylmethanderivate, Diphenylacrylatderivate, Benzimidazolderivate, p-Aminobenzoesäurederivate, Polymerfilter und Siliconfilter.

Sonnenschutzfilter, die im UV-A- und/oder UV-B-Bereich wirksam sind, können ausgewählt sein aus der Gruppe umfassend: p- Aminobenzoesäure, ethoxyliertes p-Aminobenzoat, 2-Ethylhexyl-p-dimethylaminobenzoat, N-propoxyliertes Ethyl-p-aminobenzoat, Glyceryl-p-aminobenzoat, Homomenthylsalicylat, 2-Ethylhexylsalicylat, Triethanolaminsalicylat, 4-Isopropylbenzylsalicylat, 4-tert.-Butyl-4'-methoxydibenzoylmethan, 4-Isopropyldibenzoylmethan, 2-Ethylhexyl-4-methoxycinnamat, Methyldiisopropylcinnamat, Isoamyl-4-methoxycinnamat, Diethanolamin-4-methoxycinnamat, Menthylanthranilat; 2-Ethylhexyl-2-cyano-3,3'-diphenylacrylat, Ethyl-2-cyano-3,3'-diphenylacrylat, 2-Phenylbenzimidazol-5-sulfonsäure und deren Salze, 3-(4'-Trimethylammonium)-benzyliden-bornan-2-on-methylsulfat, 1,4-Benzol-di(3-methyliden-10-camphersulfonsäure) und deren Salze, Urocansäure, 2-Hydroxy-4-methoxybenzophenon, 2-Hydroxy-4-methoxybenzophenon-5-sulfonat, 2,4-Dihydroxybenzophenon, 2,2',4,4'-Tetrahydroxybenzophenon, 2, 2'-Dihydroxy-4,4'-dimethoxybenzophenon, 2-Hydroxy-4-n-octoxybenzophenon, 2-Hydroxy-4-methoxy-4'-methylbenzophenon, α-(2-Oxoborn-3-yliden)-tolyl-4-sulfonsäure und Salze davon, 3-(4'-Sulfo)-benzyliden-bornan-2-on und Salze davon, 3-(4'-Methylbenzyliden)-d, 1-campher, 3-Benzyliden-d, 1-campher, 2,4,6-Tris-[p-(2'-ethylhexyl-1'-oxycarbonyl)-anilino]-1,3,5 -triazin, 2-[p-(tert.-Butylamido)-anilino]-4,6-bis-[(p-(2'-ethylhexyl-1'- oxycarbonyl)- anilino]-1,3,5-triazin, 1,4-bis-Benzimidazolyl-phenylen- 3,3',5,5'- tetrasulfonsäure und Salze davon, Polymer von N-[(2 und 4)- [(2-oxoborn-3-yliden)-methyl]-benzyl]-acrylamid, 2-[4-(Diethylamino)2-hydroxybenzoyl] alkylbenzoate, Polyorgano-siloxane mit Malonatgruppe.

Die erfindungsgemäß verwendbaren Zusammensetzungen mit sandabweisender Wirkung können außerdem mindestens ein Mittel zum Bräunen und/oder künstlichen Braunfärben der Haut, wie Dihydroxyaceton, enthalten.

Ferner können die erfindungsgemäßen kosmetischen Zusammensetzungen Pigmente oder auch Nanopigmente im Bereich von 5 bis 100 nm, vorzugsweise im Bereich von 10 bis 50 nm, die umhüllt oder nicht umhüllt sind, enthalten. Geeignet verwendbare Nanopigmente von Metalloxiden umfassen Oxide von Titan, beispielsweise amorph oder kristallin, in Form von Rutil und/oder Anatas, von Eisen, Mangan, Zink, Zirconium oder Cer, welche alle bekannten UV-Lichtschutzsubstanzen darstellen. Herkömmliche Umhüllungsmittel können Aluminiumoxid, Siliciumdioxid, Aluminiumstearat, Dimethicone, und/oder Methicone sein. Erfindungsgemäß verwendbare Nanopigmente von Metalloxiden, die gegebenenfalls umhüllt sind, werden beispielsweise in den Patentanmeldungen EP-A-0 518 772 und EP-A-0 518 773 beschrieben.

Weitere öllösliche W-B- und/oder Breitband-Filtersubstanzen im Sinne der vorliegenden Erfindung umfassen 3-Benzylidencampher-Derivate, vorzugsweise 3-(4-Methylbenzyliden)campher, 3-Benzylidencampher; 4-Aminobenzoesäure-Derivate, vorzugsweise 4-(Dimethylamino)benzoesäure (2-ethylhexyl)ester, 4-Dimethylamino)benzoesäureamylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie an Polymere gebundene UV-Filter wie 3-(4-(2,2-bis Ethoxycarbonylvinyl)-phenoxy)propenyl)-methoxysiloxan/Dimethylsiloxan - Copolymer.

Erfindungsgemäß verwendbare kosmetische Zusammensetzungen können 0,1 bis 20 Gew.-%, vorzugsweise 0,5 bis 15 Gew.-%, und bevorzugt 0,5 bis 10 Gew.-%, einer oder mehrerer öllöslicher UV-Filtersubstanzen aufweisen.

Die kosmetischen Zusammensetzungen können ferner 0,001 bis 15 Gew.-%, vorzugsweise 0,01 bis 10 Gew.-%, besonders bevorzugt 0,05 bis 5 Gew.-%, Zusatzstoffe und/oder kosmetische Hilfsstoffe umfassen.

Die kosmetischen Zusammensetzungen gemäß der Erfindung können ferner mindestens einen Zusatzstoff enthalten, der unter den Fettsubstanzen, den organischen Lösemitteln, den Verdickungsmitteln, den reizlindernden Mitteln, den Antioxidantien, den Trübungsmitteln, den Stabilisierungsmitteln, den Emollientien, den Hydroxysäuren, den Antischaummitteln, den Hydratisierungsmitteln, den Vitaminen, den Parfüms, den Konservierungsmitteln, den grenzflächenaktiven Stoffen, den Füllstoffen, den Maskierungsmitteln, den Polymeren, den Treibmitteln, den alkalisch machenden Mitteln oder den Säuerungsmitteln und/oder den Farbmitteln ausgewählt ist.

Die kosmetischen Zusammensetzungen gemäß der Erfindung können ferner kosmetische Hilfsstoffe enthalten, wie sie üblicherweise in solchen Zusammensetzungen verwendet werden, umfassend Konservierungsmittel, Konservierungshelfer, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Füllstoffe, die das Hautgefühl verbessern, Fette, Öle, Wachse und/oder andere übliche kosmetische Hilfsstoffe einer kosmetischen Formulierung, umfassend Alkohole, Polyole, Polymere, Schaumstabilisatoren, Elektrolyte, organische Lösungsmittel und/oder Silikonderivate.

Erfindungsgemäß bevorzugt verwendbare kosmetische Hilfsstoffe umfassen Alkohole, Ethanol und/oder Isopropanol, Diole oder Polyole sowie deren Ether, insbesondere Propylenglykol, Glycerin, Ethylenglykol, Ethylenglykolmonoethyl-oder -monobutylether, Propylenglykolmonomethyl-, -monoethyl-oder -monobutylether, Diethylenglykolmonomethyl- oder -monoethylether und analoge Produkte, Polymere, Schaumstabilisatoren, Elektrolyte, Dihydoxyaceton sowie gegebenenfalls ein oder mehrere Verdickungsmittel, wie Siliciumdioxid, Aluminiumsilikate, Polysaccharide sowie Derivate davon, Hyaluronsäure, Xanthangummi, Hydroxypropylmethylcellulose, Polyacrylate, Carbopole, beispielsweise Carbopole der Typen 980, 981, 1382, 2984, 5984, jeweils einzeln oder in Kombination. Geeignet verwendbare kosmetische Hilfsstoffe sind beispielsweise in der EP 1 555 015 0 A1 beschrieben, auf die hier in vollem Umfang Bezug genommen wird.

Die kosmetische Zusammensetzung mit sandabweisenden Esterquats gemäß der vorliegenden Erfindung kann auch Moisturizer aufweisen. Als Moisturizer werden Stoffe oder Stoffgemische bezeichnet, welche kosmetischen Zusammensetzungen die Eigenschaft verleihen, nach dem Auftragen bzw. Verteilen auf der Hautoberfläche die Feuchtigkeitsabgabe der Hornschicht zu reduzieren und/oder die Hydratation der Hornschicht positiv zu beeinflussen.

Verwendbare Moisturizer umfassen Glycerin, Milchsäure und/oder Lactate, insbesondere Natriumlactat, Butylenglykol, Propylenglykol, Biosaccaride, Glycine, Soja, Ethylhexyloxyglycerin, Pyrrolidoncarbonsäure und Harnstoff, sowie polymere Moisturizer aus der Gruppe der wasserlöslichen und/oder in Wasser quellbaren und/oder mit Hilfe von Wasser gelierbaren Polysaccharide. Geeignet sind ferner Hyaluronsäure, Chitosan und/oder ein fucosereiches Polysaccharid, welches in den Chemical Abstracts unter der Registraturnummer 178463-23-5 abgelegt und beispielsweise unter der Bezeichnung Fucogel®1000 von der Gesellschaft SOLABIA S.A. erhältlich ist.

Verwendbare Konservierungsmittel im Sinne der vorliegenden Erfindung umfassen Formaldehydabspalter, Iodopropylbutylcarbamate, Parabene, p-Hydroxybenzoesäurealkylester, wie Methyl-, Ethyl-, Propyl- und/oder Butylparaben, Phenoxyethanol, Ethanol, Benzoesäure. Üblicherweise umfasst das Konservierungssystem erfindungsgemäß ferner vorteilhaft auch Konservierungshelfer, wie beispielsweise Octoxyglycerin, Glycine, und/oder Soja.

Verwendbare Antioxidatien im Sinne der vorliegenden Erfindung umfassen wasserlösliche Antioxidantien, Vitamine, insbesondere Ascorbinsäure und deren Derivate. Bevorzugte Antioxidantien sind ferner Vitamin E und dessen Derivate sowie Vitamin A und dessen Derivate.

Die Menge der Antioxidantien, bezogen auf das Gesamtgewicht der kosmetischen Zusammensetzung kann 0,001 bis 30 Gew.- %, bevorzugt 0,05 bis 15 Gew.-%, insbesondere 0,5 bis 10 Gew.-%, ausmachen.

Verwendbare natürliche Wirkstoffe und/oder deren Derivate umfassen alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin.

Die kosmetische Zusammensetzung mit sandabweisenden Esterquats kann auch Füllstoffe enthalten, welche die sensorischen und kosmetischen Eigenschaften der Formulierungen weiter verbessern und beispielsweise ein samtiges oder seidiges Hautgefühl hervorrufen oder verstärken. Verwendbare Füllstoffe umfassen Stärke und Stärkederivate, wie Tapiocastärke, Distärkephosphat, Aluminium- bzw. Natrium-Stärke, Octenylsuccinat, Pigmente, die weder hauptsächlich UV-Filter- noch färbende Wirkung haben, beispielsweise Bornitrid und/oder Aerosile® (CAS-Nr. 7631-86-9).

Ferner kann es gegebenenfalls von Vorteil sein, Filmbildner in die erfindungsgemäßen kosmetischen Zusammensetzungen einzuarbeiten, beispielsweise um die Wasserfestigkeit der Zusammensetzungen zu verbessern oder die UV-Schutzleistung zu erhöhen. Geeignet sind sowohl wasserlösliche, dispergierbare und/oder fettlösliche Filmbildner, jeweils einzeln oder in Kombination miteinander.

Erfindungsgemäß verwendbare Filmbildner umfassen Polyurethane, Dimethicone, Copolyole, Polyacrylate, PVP/VA Copolymer (PVP = Polyvinylpyrrolidon, VA = Vinylacetat).

Fettlösliche Filmbildner können ausgewählt sein aus der Gruppe der Polymere auf Basis von Polyvinylpyrrolidon (PVP), Copolymere des Polyvinylpyrrolidons, PVP/Hexadecen-Copolymer und/oder das PVP/Eicosen-Copolymer.

Die Erfindung wird anhand der nachfolgenden Beispiele 1 bis 8 verdeutlicht, ohne sie einzuschränken. Die Zahlenwerte in den Beispielen, wenn nicht anders angegeben, bedeuten Gewichtsprozente, bezogen auf das Gesamtgewicht der jeweiligen Zusammensetzungen.

### Allgemeine Testmethode

Es wurden 0,3 g einer Emulsion der nachstehenden Beispiele 1 bis 8 auf der Innenseite von trockenen, schweißfreien Unterarmen von Testpersonen im Alter von 30 bis 50 Jahren auf 150 cm³ aufgetragen, wobei die bei Inaugenscheinnahme jeweiligen trockenen Unterarme bei Raumtemperatur zusätzlich 10 min gelüftet und bis zum Einziehen der Emulsion 2 Minuten homogen eingerieben wurden. Anschließend wurden die Unterarme, an denen die Emulsion aufgetragen wurde, auf der Innenseite mit 30 ml feinem Aquariensand, erhältlich bei der Fa. Interseroh, Artikel-Nr. 388 2849, über einen Zeitraum von 15 Sekunden übergossen, wobei der auf Raumtemperatur abgekühlte Sand vorher über einen Zeitraum von 48 Stunden in einem Trocknungsofen bei einer Temperatur von 80° C getrocknet wurde. Durch dreimaliges kräftiges Klatschen in die Hände wurde loser anhaftender Sand entfernt. Anschließend wurde der an der Unterarminnenfläche zurückbleibende anhaftende Sand mit Ethanol vollständig abgespült und in einem Becherglas aufgefangen. Das Ethanol wurde entfernt und der im Becherglas zurückbleibende Sand ausgewogen.

**Tabelle 1:**

| Zusammensetzung der Beispiele 1 bis 8 | | | | | | | | |
|---|---|---|---|---|---|---|---|---|
| Zusammensetzung | 1 | 2 | 3 | 4 | 5 | 6 | 7 | 8 |
| Rewoquat® WE 15*¹ | 3,5 | | | | 3,5 | | | |
| Rewoquat® WE 28*² | | 3,5 | | | | 3,5 | | |
| Rewoquat® WE 38 DPG*³ | | | 3,5 | | | | 3,5 | |
| Rewoquat® WE 18-E*⁴ | | | | 3,5 | | | | 3,5 |
| Abil® Quat 3272*⁵ | | | | | 1,0 | 1,0 | 1,0 | 1,0 |
| Tegin® M*⁶ | 1,5 | 1,5 | 1,5 | 1,5 | 1,5 | 1,5 | 1,5 | 1,5 |
| Tego® Alkanol 18*⁷ | 1,0 | 1,0 | 1,0 | 1,0 | 1,0 | 1,0 | 1,0 | 1,0 |
| Tegosoft® TN*⁸ | 7,0 | 7,0 | 7,0 | 7,0 | 7,0 | 7,0 | 7,0 | 7,0 |
| Tegosoft® DEC*⁹ | 3,0 | 3,0 | 3,0 | 3,0 | 3,0 | 3,0 | 3,0 | 3,0 |
| Tegosoft® CR*¹⁰ | 1,0 | 1,0 | 1,0 | 1,0 | 1,0 | 1,0 | 1,0 | 1,0 |
| Tegosoft® TIS*¹¹ | 1,0 | 1,0 | 1,0 | 1,0 | | | | |
| BMDM*¹² | 3,0 | 3,0 | 3,0 | 3,0 | 3,0 | 3,0 | 3,0 | 3,0 |
| EHMC*¹³ | 5,0 | 5,0 | 5,0 | 5,0 | 5,0 | 5,0 | 5,0 | 5,0 |
| OC*¹⁴ | 3,0 | 3,0 | 3,0 | 3,0 | 3,0 | 3,0 | 3,0 | 3,0 |
| Glycerin | 3,0 | 3,0 | 3,0 | 3,0 | 3,0 | 3,0 | 3,0 | 3,0 |
| Wasser | 68,0 | 68,0 | 68,0 | 68,0 | 68,0 | 68,0 | 68,0 | 68,0 |
| Gew.-% | 100 | 100 | 100 | 100 | 100 | 100 | 100 | 100 |

| | | | | | | | | |
|---|---|---|---|---|---|---|---|---|
| Rewoquat® WE 15*¹: Di-(oleylcarboxyethyl)-hydroxyethyl-methylammonium-methosulfat erhältlich bei der Firma Goldschmidt Rewo GmbH & Co. KG. Rewoquat® WE 28*²: Di-(palmoyl)-hydroxyethyl-methylammonium-methosulfat + Isopropanol erhältlich bei der Firma Goldschmidt Rewo GmbH & Co. KG. Rewoquat® WE 38 DPG*³: Di-(palmoyl)-hydroxyethyl-methylammonium-methosulfat + Dipropylenglycol erhältlich bei der Firma Goldschmidt Rewo GmbH & Co. KG. Rewoquat® WE 18-E*⁴: Di-(palmoyl)-hydroxyethyl-methylammonium-methosulfat + Ethanol erhältlich bei der Firma Goldschmidt Rewo GmbH & Co. KG. Abil® Quat 3272*⁵: alpha-omega-quaterniertes Polydimethylsiloxan, erhältlich bei der Firma Goldschmidt GmbH. Tegin® M*⁶: Glycerinstearat, erhältlich bei der Firma Goldschmidt GmbH. Tego® Alkanol 18*⁷: C₁₈-Alkohol, erhältlich bei der Firma Goldschmidt GmbH. Tegosoft® TN*⁸: C₁₂-C₁₅-Alkylbenzoat, erhältlich bei der Firma Goldschmidt GmbH. Tegosoft® DEC*⁹: Dihexylethylcarbonat, erhältlich bei der Firma Goldschmidt GmbH. Tegosoft® CR*¹⁰: Cetylrizinoleat, erhältlich bei der Firma Goldschmidt GmbH. Tegosoft® TIS*¹¹: Triisostearin, erhältlich bei der Firma Goldschmidt GmbH. BMDM*¹²: Butylmethoxydibenzoylmethan. EHMC*¹³: Ethylhexylmethoxycinnamat. OC*¹⁴: Octocrylen. | | | | | | | | |

Die Zusammensetzungen der Beispiele 1 bis 8 wurden durch Mischen der jeweiligen Komponenten, wie in Tabelle 1 angegeben, erhalten.

Die erfindungsgemäßen sandabweisenden Zusammensetzungen der Beispiele 1 bis 8 wurden jeweils gemäß der vorstehend beschriebenen allgemeinen Testmethode bei jeweils 10 Testpersonen der Altersgruppe 30 bis 50 Jahre an der Unterarminnenfläche aufgetragen und die Sandanhaftung, wie beschrieben, bestimmt, wobei aus den jeweiligen 10 Sandanhaftungsversuchen für jede kosmetische Zusammensetzung der Beispiele 1 bis 8 der jeweilige Mittelwert errechnet wurde.

Als Vergleichsversuch wurden 10 weitere Testpersonen gemäß der vorstehend beschriebenen allgemeinen Testmethode mit einem Sonnenschutzmittel Ombra® erhältlich bei ALDI Süd (Aldi Einkauf GmbH & Co. oHG, Essen, Deutschland) behandelt und die Sandanhaftung bestimmt. Die jeweils gemittelten Ergebnisse sind in der Tabelle 2 für die Beispiele 1 bis 8 und das Vergleichsbeispiel bewertet.

**Tabelle 2:**

| Beispiele | Sandabweisende Wirkung |
|---|---|
| 1 | + |
| 2 | ++ |
| 3 | +++ |
| 4 | ++++ |
| 5 | + |
| 6 | +++ |
| 7 | ++ |
| 8 | ++++ |
| Sonnenschutzmittel Ombra® | -- |

| | |
|---|---|
| + = gute sandabweisende Eigenschaften < 1 g ++ bis +++ = sehr gute sandabweisende Eigenschaften < 0,5 g ++++ = beste sandabweisende Eigenschaften < 0,3 g - = schlechte sandabweisende Eigenschaften > 1g -- = sehr schlechte sandabweisende Eigenschaften > 1,5 g | |

Das jeweils beste Ergebnis für die Beispiele 1 bis 8 und das Vergleichsbeispiel sind in der Tabelle 3 wiedergeben.

**Tabelle 3:**

| Beispiele | Sandabweisende Wirkung, als Sandrückstand in [g], ermittelt nach Entfernen des Alkohols |
|---|---|
| 1 | < 0,9 |
| 2 | < 0,6 |
| 3 | < 0,55 |
| 4 | < 0,25 |
| 5 | < 1,1 |
| 6 | < 0,6 |
| 7 | < 0,8 |
| 8 | < 0,4 |
| Sonnenschutzmittel Ombra^{®} | > 1,6 |

### Siebanalyse

Die jeweils verwendeten Chargen des feinen Aquariensands, erhältlich bei der Fa. Interseroh, Artikel-Nr. 388 2849, wurden vorab einer Siebanalyse unterzogen, die nachstehend angegeben ist:
26 Gew.-% - 34 Gew.-% weisen eine Partikelgröße von < 0,315 mm,
42 Gew.-% - 50 Gew.-% weisen eine Partikelgröße von > 0,315 mm,
19 Gew.-% - 24 Gew.-% weisen eine Partikelgröße von > 0,5 mm,
0,5 Gew.-% - 3 Gew.-% weisen eine Partikelgröße von > 0,8 mm.

Es versteht sich von selbst, dass der Aquariensand trotz unterschiedlicher Partikelgrößenverteilung der jeweiligen verwendeten Chargen je Charge ein Gesamtgewicht von 100 Gew.-% hat.

Auf die in dieser Beschreibung aufgeführten Literaturstellen und Patentanmeldungen/Patente wird in vollem Umfang Bezug genommen und sie sind vollständiger, inhaltlicher Bestand dieser Anmeldung.

## Patentansprüche

1. Verwendung wenigstens eines Esterquats auf Basis von Alkanolaminen in Zusammensetzungen, wobei die Esterquats für die Zusammensetzung eine sandabweisende Wirkung hervorrufen, **dadurch gekennzeichnet, dass** die Acylkomponente des Esterquats sich von
(a) Monocarbonsäuren,
(b) Dicarbonsäuren,
(c) Tricarbonsäuren,
oder deren Gemischen ableitet.

2. Verwendung nach Anspruch 1, **dadurch gekennzeichnet, dass** die Esterquats die nachstehende Formel I, Formel II und/oder Formel III aufweisen: 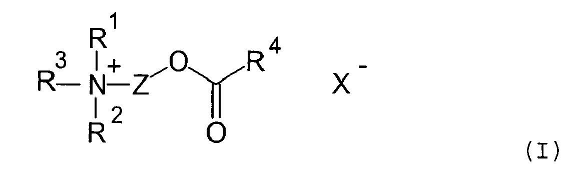 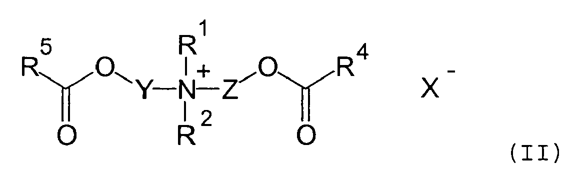 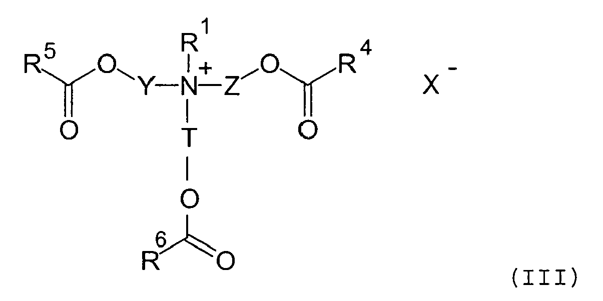 in der bedeuten:
R¹ = H, Methyl, Ethyl, Propyl oder iso-Propyl,vorzugsweise Methyl oder Ethyl,
R² R³ = gleich oder unabhängig voneinander, R¹, verzweigter oder unverzweigter, gesättigter oder ungesättigter Hydroxy-alkylrest mit C₁-C₆ Kohlenstoffatomen, vorzugsweise verzweigtes oder unverzweigtes C₃-C₅-Hydroxyalkyl, verzweigtes oder unverzweigtes C₃-C₅-Hydroxyalkenyl, und besonders bevorzugt Hydroxyethyl oder Hydroxyisopropyl,
R⁴, R⁵ und R⁶ = gleich oder unabhängig voneinander, verzweigter oder unverzweigter, gesättigter oder ungesättigter Acyl-Rest mit C₅-C₂₃ Kohlenstoffatomen, vorzugsweise mit C₇-C₂₁ Kohlenstoffatomen, besonders bevorzugt mit C₁₁-C₁₉ Kohlenstoffatomen und am meisten bevorzugt mit C₁₅-C₁₇ Kohlenstoffatomen,
T, Y und Z = gleich oder unabhängig voneinander, Methylen, Ethylen, Ethenylen, verzweigtes oder unverzweigtes, gesättigtes oder ungesättigtes Alkylen mit C₃-C₅ Kohlenstoffatomen, und
X = Anion, vorzugsweise ist das Anion ein Halogenid, Alkylsulfat oder Alkylphosphat, und besonders bevorzugt ist das Anion Cl⁻, CH₃OSO₃⁻ oder CH₃CH₂OSO₃⁻.

3. Verwendung nach Anspruch 1 oder 2, **dadurch gekennzeichnet, dass** das/die Esterquat/s ausgewählt ist/sind aus der Gruppe umfassend Di-(oleylcarboxyethyl)-hydroxyethyl-methylammoniumsalz, Di-(talgcarboxyethyl)-hydroxyethyl-methylammoniumsalz, N,N-Di-(ß-stearoylethyl)-N,N-dimethyl-ammoniumsalz, N,N-Di-(ß-palmitoylethyl)-N,N-dimethyl-ammoniumsalz, Dicocoylethyl-hydroxyethylammonium-methosulfate, Dipalmoylethyl-hydroxyethylammoniummethosulfate, Dirapscarboxyethyl- hydroxyethylammoniummethosulfate, und/oder Disoyacarboxyethyl-hydroxyethylammonium-methosulfate und/oder deren hydrierte Analoga.

4. Verwendung nach einem der Ansprüche 1 bis 3, **dadurch gekennzeichnet, dass** das/die Esterquat/s einen durchschnittlichen Veresterungsgrad von 1 bis 3, vorzugsweise 1,5 bis 2,5 und bevorzugt 1,7 bis 2,2 aufweist/aufweisen.

5. Verwendung nach einem der Ansprüche 1 bis 4, **dadurch gekennzeichnet, dass** das/die Esterquat/s eine Jodzahl von ≥ 20 bis ≤ 100, vorzugsweise eine Jodzahl von ≥ 30 bis ≤ 90, und besonders bevorzugt eine Jodzahl von ≥ 40 bis ≤ 80, aufweist/aufweisen.

6. Kosmetische Zusammensetzung zur topischen Verwendung mit sandabweisender Wirkung, **dadurch gekennzeichnet, dass** die kosmetische Zusammensetzung einen kosmetisch akzeptablen Träger und wenigstens ein Esterquat nach einem der Ansprüche 1 bis 5 als sandabweisende Substanz aufweist.

7. Kosmetische Zusammensetzung nach Anspruch 6, **dadurch gekennzeichnet, dass** die kosmetische Zusammensetzung Esterquat/s als sandabweisende Substanz mit einem Gewichtsgehalt, bezogen auf das Gesamtgewicht der kosmetischen Zubereitung, von 0,1 Gew.-% bis 10 Gew.-%, vorzugsweise von 1 Gew.-% bis 8 Gew.-%, und besonders bevorzugt von 2 Gew.-% bis 4 Gew.-%, aufweist.

8. Kosmetische Zusammensetzung nach einem der Ansprüche 6 oder 7, **dadurch gekennzeichnet, dass** der kosmetisch akzetable Träger in Form einer Emulsion vom Öl-in-Wasser-Typ vorliegt.

9. Kosmetische Zusammensetzung nach einem der Ansprüche 6 bis 8,**dadurch gekennzeichnet, dass** sie außerdem ein oder mehrere ergänzende hydrophile oder lipophile, im UV-A- und/oder UV-B-Bereich wirksame organische Filter, und/oder Pigmente oder Nanopigmente von Metalloxiden, die umhüllt oder nicht umhüllt sind, enthält.

10. Kosmetische Zusammensetzung nach einem der Ansprüche 6 bis 9, **dadurch gekennzeichnet, dass** sie außerdem mindestens ein Mittel zum Bräunen und/oder künstlichen Braunfärben der Haut enthält.

11. Kosmetische Zusammensetzung nach einem der Ansprüche 6 bis 10, **dadurch gekennzeichnet, dass** sie außerdem mindestens einen kosmetischen Hilfsstoff und/oder Zusatzstoff enthält, der unter den Fettsubstanzen, den organischen Lösemitteln, den Verdickungsmitteln, den reizlindernden Mitteln, den Antioxidantien, den Trübungsmitteln, den Stabilisierungsmitteln, den Emollientien, den Hydroxysäuren, den Antischaummitteln, den Hydratisierungsmitteln, den Vitaminen, den Parfüms, den Konservierungsmitteln, den grenzflächenaktiven Stoffen, den Füllstoffen, den Maskierungsmitteln, den Polymeren, den Treibmitteln, den alkalisch machenden Mitteln oder den Säuerungsmitteln, und/oder den Farbmitteln ausgewählt ist.

12. Kosmetische Zusammensetzung nach einem der Ansprüche 6 bis 11, **dadurch gekennzeichnet, dass** es sich um eine Zusammensetzung zum Schutz der menschlichen Epidermis oder ein Sonnenschutzmittel handelt und dass sie in Form einer nicht-ionischen Vesikeldispersion, einer Emulsion, insbesondere einer Emulsion vom Öl-in-Wasser-Typ, einer Creme, einer Milch, eines Gels, einer Gelcreme, einer Suspension, einer Dispersion, eines Puders, eines festen Stifts, eines Schaumes oder eines Sprays vorliegt.

13. Kosmetische Zusammensetzung nach einem der Ansprüche 6 bis 12, **dadurch gekennzeichnet, dass** es sich um eine Zusammensetzung zum Schminken der Wimpern, der Augenbrauen oder Haut handelt, und dass sie in fester oder pastöser, wasserfreier oder wässriger Form, in Form einer Emulsion, einer Suspension oder einer Dispersion vorliegt.

14. Verfahren zur topischen kosmetischen Behandlung der Haut zwecks Erzielung einer sandabweisenden Wirkung sowie gegebenenfalls zusätzlich zum Schutz vor Ultraviolett-Strahlung, insbesondere Sonnenlicht, **dadurch gekennzeichnet, dass** es darin besteht, auf die Haut eine wirksame Menge einer Zusammensetzung aufzutragen, die wie in einem der Ansprüche 1 bis 13 definiert ist.
